# NOUVEAU FASCICULE DE BREVET EUROPEEN

(11) **EP 0 873 687 B2**
(45) Date de publication et mention de la décision concernant l'opposition: **26.04.2006**
(45) Mention de la délivrance du brevet: 05.06.2002
(21) Numéro de dépôt: 98400851.6
(22) Date de dépôt: 08.04.1998
(51) Int. Cl.: A01N 37/16, A01N 33/24, A01N 25/30

(54) **Composition désinfectante et fongicide à base d'acide péracétique et d'un oxyde d'amine**
Desinfizierende und fungizide Zusammensetzung auf der Basis von Peressigsäure und einem Aminoxid
Disinfecting and fungicidal composition based on peracetic acid and an amine oxide

(30) Priorité: 24.04.1997 FR 9705052; 24.12.1997 FR 9716490
(43) Date de publication de la demande: 28.10.1998
(73) Titulaire: CHEMOXAL SA, F-75007 Paris (FR); SOCIETE D'EXPLOITATION DE PRODUITS POUR LES INDUSTRIES CHIMIQUES, S.E.P.P.I.C., 75321 Paris Cédex 07 (FR)
(72) Inventeur: Hamon Godin, Catherine, Singapore 118756 (SG); Gouges, Yves, 75014 Paris (FR); Le Rouzic, Daniel, 95120 Ermont (FR)
(74) Mandataire: Conan, Philippe Claude

(56) Documents cités:
- WO-A-96/19558
- WO-A-96/19559
- DE-A- 2 536 617
- DE-A- 4 331 942
- GB-A- 2 255 507
- Roempp Lexikon Chemie, 10e. Edition, Georg Thieme Verlag
- Römpp Lexikon Chemie, 10e. Edition, Georg Thieme Verlag

## Description

L'invention concerne de nouvelles compositions désinfectantes contenant de l'acide péracétique.

De telles solutions sont aujourd'hui largement utilisées dans de nombreux secteurs de l'industrie. L'intérêt croissant de l'acide peracétique réside dans sa large gamme d'activité biocide, en particulier vis-à-vis des bactéries, des algues, des levures, des moisissures, des champignons et des virus. Néanmoins, les solutions d'acide peracétique diluées présentent des performances de mouillage insuffisantes sur les surfaces couramment traitées, par exemple dans le domaine médical et hospitalier, dans les industries agro-alimentaires, pharmaceutiques, cosmétiques, micro-électroniques, les bio-industries, les collectivités et, de façon plus générale, dans toutes les activités pour lesquelles un contrôle de la population des micro-organismes est nécessaire. Les surfaces à traiter sont très diverses ; on peut citer par exemple, les locaux, les équipements, les matériels, les outils et ustensiles, les contenants, tels que les cuves, les emballages de conditionnement, les tuyaux. On a donc cherché à abaisser la tension superficielle de ces solutions désinfectantes par l'addition d'agents tensioactifs. Parmi les documents de l'état de la technique, on peut notamment citer ceux qui décrivent des compositions comportant un peracide et un agent tensioactif non ionique comme les demandes internationales de brevet publiées sous les numéros WO 88108667, WO 93/10088 et WO 94/14321 ou les demandes de brevet européen EP 193 416 et EP 596 493.

Cependant, aucune des solutions désinfectantes à base d'acide peracétique décrites dans cet état de la technique, ne possède l'ensemble des propriétés nécessaires pour être commercialement acceptable ; ces propriétés sont les suivantes : la solution doit contenir à la fois l'acide peracétique et l'agent tensioactif, elle doit être stable dans le temps, elle doit posséder un fort pouvoir mouillant et un faible pouvoir moussant, elle doit être aisément rinçable, avoir un aspect limpide, elle doit contenir des quantités minimales de produits chimiques, tout en étant suffisamment active après dilution vis-à-vis des micro-organismes, et enfin le ou les agents tensioactifs utilisés doivent être compatibles avec l'acide peracétique. De plus, lorsqu'il s'agit de désinfecter et de stériliser du matériel, il est indispensable que la solution désinfectante ait une activité fongicide améliorée. Cette exigence est incontournable lorsque l'on s'intéresse à des activités médico-chirurgicales comme par exemple, l'endoscopie, tant interventionnelle qu'exploratrice, qui est en plein essor. En effet, on peut donc craindre qu'une multiplication des gestes endoscopiques ne s'accompagne d'une multiplication des infections post-endoscopies. C'est pourquoi, les procédures de désinfection à appliquer aux équipements médicaux réutilisables sont d'une grande importance tant pour la protection des patients que celle du personnel des soins contre les risques infectieux.

Or, parmi les équipements médicaux réutilisables, les endoscopes souples sont particulièrement onéreux. Compte tenu du nombre limité de ces équipements à l'hôpital, leur réutilisation est de plus en plus fréquente et par conséquent le délai entre deux interventions est de plus en plus court. C'est pourquoi, ces pratiques engendrent de nouvelles procédures de désinfection se caractérisant principalement par leur simplicité, leur rapidité et leur coût réduit.

Cependant, l'utilisation de l'acide peracétique seule, comme agent fongicide, nécessite des doses efficaces élevées. Le plus souvent, les solutions manquent de rémanence. D'où l'intérêt de les renforcer par ajout d'un agent fongicide en vue d'augmenter la longévité des bains désinfectants et de diminuer les doses efficaces à mettre en oeuvre.

Les principes actifs antimicrobiens disposent de différents modes d'action. D'une façon générale, ils agissent, soit sur les constituants intracellulaires (enzymes, mitochondries, ...), soit extracellulaires (membrane cytoplasmique et paroi cellulaires). Les composés qui agissent à l'intérieur de la cellule doivent pour atteindre leur cible, traverser la barrière que constitue la membrane cytoplasmique, d'où l'intérêt d'agir sur cette dernière. Les ammoniums quaternaires sont reconnus comme disposant d'un excellent pouvoir antimicrobien, ceci est dû au fait qu'ils sont tensioactifs et chargés positivement. La membrane cellulaire étant chargée négativement, ils peuvent y être absorbés rapidement et s'y fixer, ce qui va désorganiser la membrane cytoplasmique, modifier la perméabilité cellulaire, précipiter les constituants cellulaires entraînant une mort rapide de la cellule même à des doses faibles. Les composés chargés positivement et de poids moléculaire élevé s'avèrent donc être dignes d'intérêt (bétaïnes, sels de phosphonium, zwitterions, ammoniums quaternaires, etc...). Il existe de nombreuses références sur l'exploitation industrielle des ammoniums quaternaires. Les amines oxydes sont des composés intéressants car mis en solution acide, ils deviennent cationiques.

Les amines oxydes ont fait l'objet de nombreuses études. Ils sont en général biodégradables et s'avèrent être des constituants très efficaces dans les détergents et les shampooings mais également en désinfection. On peut citer comme référence la revue de S. H. SHAPIRO publiée pages 32 à 50 dans le supplément de la 2^{ème} édition du Kirk Othmer's Encyclopedy of Chemical Technology.

Les oxydes d'amines n'ont été, jusqu'à présent, utilisés que comme co-agents de surface dans des compositions épaissies. On peut par exemple citer à ce sujet les demandes internationales de brevet publiées sous les numéros WO 96/19558, WO 96/19559, WO 96/17044, le brevet américain n°5,078, 896 et la demande de brevet britannique n°GB 2 255 507. La demande de brevet allemand DE 4331942 divulgue une composition nettoyante préparée à partir de 53 % en poids d'eau, 12 % en poids d'acide acétique, 2 % en poids d'acide phosphorique, 30 % en poids de peroxyde d'hydrogène et 3 % en poids de coconut alkyldiméthylamine oxyde.

Or, la demanderesse a trouvé, de façon inattendue, que les oxydes d'amines agissaient à pH acide en synergie avec l'acide peracétique, à des concentrations en solution qui n'entraînent pas d'épaississement notable de ladite solution et qui n'en détériorent pas la stabilité.

La présente invention a donc pour objet une composition telle que définie à la revendication 1.

Lorsque R₁ représente un radical acyclique, il s'agit notamment d'un radical alkyle linéaire ou ramifié ; ce radical peut être non substitué, mono- substitué ou polysubstitué, lorsque ce radical est substitué, le ou les substituants sont choisis indépendamment l'un de l'autre parmi les radicaux hydroxy, alkyloxy, alkylthio, carboxy, alkoxycarbonyle, parmi les atomes d'halogène, ou parmi les radicaux cycloalkyle ou phényle ; lorsque R₁ représente un radical cyclique, il s'agit d'un radical mononucléaire ou polynucléaire; ce radical peut être non substitué, monosubstitué ou polysubstitué ; lorsque ce radical est substitué, le ou les substituants sont choisis indépendamment l'un de l'autre parmi les radicaux hydroxy, alkyloxy, alkylthio, carboxy, alkoxycarbonyle, parmi les atomes d'halogène, ou parmi les radicaux cycloalkyle ou phényle ou encore parmi les radicaux alkyle ; lorsque R₁ représente un radical mononucléaire, il s'agit d'un radical phényle ou cycloalkyle ; lorsque R₁ représente un radical polynucléaire, il s'agit notamment de bicyles saturés, aromatiques, ou comportant un noyau aromatique ; lorsque R₁ et R₂ représentent, avec l'atome d'azote auquel ils sont liés, un hétérocyle, il s'agit d'un radical mononucléaire, saturé, ou aromatique ou d'un radical polynucléaire, saturé, aromatique ou comportant un noyau aromatique ; ce radical mononucléaire ou polynucléaire peut être non substitué, mono-substitué
ou polysubstitué ; lorsque ce radical est substitué, le ou les substituants sont choisis indépendamment l'un de l'autre parmi les radicaux hydroxy, alkyloxy, alkylthio, carboxy, alkoxycarbonyle, parmi les atomes d'halogène, ou parmi les radicaux cycloalkyle ou phényle ou encore parmi les radicaux alkyle.

A titre d'exemple et de manière non limitative, on peut citer les composés de formule (I) telle que définie précédemment, dans laquelle R₁ représente un radical méthyle, éthyle, propyle, butyle, phényle, tolyle, benzyle, octyle, nonyle, décyle, undécyle, dodécyle, tridécyle, tétradécyle, pentadécyle, hexadécyle, heptadécyle, octadécyle, imidazolinyle, morpholinyle, pipéridinyle, oxazolinyle, pipérazinyle, pentyle, hexyle, heptyle, norbomyle, cyclopentyle, cyclohexyle, thiazolyle, oxazolyle, naphtyle, tétrahydronaphtyle, phénétyle, pyridyle, quinolyle, carboxy 1-pyridyle, méthoxycarbonyl 1-pyridyle ou éthoxycarbonyl 1-pyridyle, ou les composés de formule (I) dans laquelle R₁ et R₂ représentent avec l'atome auquel ils sont liés un radical imidazolinyle, morpholinyle, pipéridinyle, oxazolinyle, pipérazinyle, pyridyle, quinolyle, carboxy 1-pyridyle, méthoxycarbonyl 1-pyridyle, thiazolyle ou oxazolyle.

De tels composés de formule (I) peuvent être préparés selon des méthodes connues de l'homme du métier à partir de l'amine correspondante,
ou sont disponibles dans le commerce sous la marque AROMOX® comme le composé AROMOX® MCD-W.

Par au moins un composé de formule (I), on signifie qu'il peut s'agir soit d'un composé de formule (I) unique, soit d'un mélange de composés de formules (I); dans ce dernier cas, il est bien entendu que les pourcentages pondéraux indiqués s'appliquent au mélange dans son ensemble.

Par au moins un agent tensioactif non ionique, on désigne un ou plusieurs agents de surface qui ne s'ionisent pas ou peu en solution aqueuse. On peut citer par exemple les alcools gras alkoxylés.

Comme agent tensioactif non ionique composé de formule (II) approprié à la présente invention, il y a notamment le Génapol®2822, le Génapol®2908D, le Génapol®2909D, ou le Simulsol® NW 900 qui sont tous des produits disponibles dans le commerce et dont la composition chimique est la suivante :

| | |
|---|---|
| **Nom** | **Composition chimique du tensioactif** |
| Génapol® 2822 | mélange d'alcools alkoxylés en C_{10'} C_{12'} C_{14'} (50E, 4 OP) |
| Génapol® 2908D | mélange d'alcools alkoxylés en C_{11'} C_{13'} C_{15'} (6 à 7 OE, 3 OP) |
| Génapol® 2909 | mélange d'alcools alkoxylés en C_{12'} et C_{14'} (50E, 3 OP) |
| Simulsol® NW 900 | alcools alcoxylés (x OE, y OP) |

Par au moins un composé de formule (II), on signifie qu'il peut s'agir, soit d'un composé de formule (II), soit d'un mélange de composés de formules (II) ; dans ce dernier cas, il est bien entendu que les pourcentages pondéraux s'appliquent au mélange dans son ensemble.

Dans la définition de la formule (II), le groupe [CH(R₈)-CH(R₆)-O]ₙ signifie qu'il peut s'agir d'une chaîne composée, soit uniquement de groupes éthoxyle (R₆ et R₈=H), soit uniquement de groupes propoxyle (R₆ ou R₈=CH₃), soit uniquement de groupes butoxyle (R₆ ou R₈=CH₂-CH₃), soit un mélange de deux sortes ou des trois sortes de groupes.

Dans ce dernier cas, les différents fragments sont distribués de manière séquencée ou aléatoire.

L'invention a notamment pour objet la composition telle que définie précédemment pour laquelle dans la formule (1), R₁ représente un radical aliphatique, linéaire ou ramifié, comportant de 8 à 18 atomes de carbone et R₂ et R₃ représentent chacun un radical méthyle.

Dans une première variante préférée de la présente invention, dans la formule (I), R₁ est choisi parmi les radicaux octyle, décyle, dodécyle, tétradécyle ou hexadécyle.

Comme composé de formule (I) approprié à la présente invention, il y a notamment l'oxyde de cocodiméthylamine, l'oxyde de myristamine, l'oxyde de dihydroxyéthyl cocoamine, l'oxyde de diméthyl stéarylamine ou l'oxyde de diéthyl stéarylamine.

Dans une deuxième variante préférée de la présente invention, dans la formule (II), R₇ représente un atome d'hydrogène.

Dans une troisième variante préférée de la présente invention, dans la formule (II), soit le groupe [CH(R₈)-CH(R₆)-o]ₙ représente uniquement des motifs structuraux [CH₂-CH₂-O]ₙ soit le groupe [CH(R₈)-CH(R₆)-O]ₙ représente une chaîne constituée environ d'entre 5 et 8 motifs structuraux -(CH₂-CH₂-O)- et environ d'entre 1 et 5 motifs structuraux -(CH₂₋CH(CH₃)-O), lesdits motifs structuraux étant distribués dans la dite chaîne de manière séquencée ou aléatoire.

Les solutions aqueuses selon l'invention peuvent comporter en outre, les composés classiques connus de l'homme du métier tels que des stabilisants ; de tels composés sont, en général, présents dans la solution d'acide peracétique et de peroxyde d'hydrogène utilisée pour la préparation des compositions selon l'invention ; ce sont aussi par exemple les acides forts ou leurs sels alcalins, les agents séquestrants ou les capteurs de radicaux libres ; on peut citer l'acide sulfurique, l'acide phosphorique, le pyrophosphate de sodium ou encore l'acide dipicolinique, les acides phosphoniques ou les butyl hydroxy toluènes. Selon l'utilisation envisagée, la composition selon l'invention peut aussi contenir des colorants et/ou des parfums.

La solution aqueuse selon l'invention peut aussi comporter un agent de coloration tel qu'un colorant notamment un composé comportant une fonction monoazo -N = N-, ou un pigment commercialisé sous forme de dispersion prête à l'emploi notamment de la famille des phtalocyanines.

L'invention a notamment pour objet une composition telle que définie précédemment, sous forme de solution aqueuse comprenant de 0,0001 % à 1 % en poids d'un agent de coloration. Par agent de coloration, en entend tout agent comptible avec l'acide peracétique et notamment ceux décrits dans la demande de brevet européen EP0658309 publiée le 21 juin 1995 et notamment les composés suivants l'Orange soleil W2002 ou le Rouge vif W3002, commercialisés par la société WACKER; l'HOSTAFINE® Bleu B26, ou l'HOSTAFINE® vert GN commercialisés par la société HOECHST AG ; et le colorant E102.

Selon l'utilisation qui est préconisée, la composition selon l'invention sera plus ou moins concentrée en acide peracétique.

De telles compositions prêtes à l'emploi présentent de préférence un rapport pondéral (acide peracétique + acide acétique) / peroxyde d'hydrogène qui est inférieur à 1.

La composition selon l'invention est préparée selon des méthodes connues de l'homme du métier notamment à partir de solutions et d'agents tensioactifs commerciaux ou décrits dans la littérature. On peut par exemple, soit ajouter le produit de formule (I) à une solution aqueuse comprenant de l'acide peracétique, du peroxyde d'hydrogène et de l'acide acétique ; soit ajouter l'amine tertiaire correspondant au produit de formule (I) dans ladite solution ; le composé de formule (I) est alors préparé « in situ » par action du peroxyde d'hydrogène sur l'amine tertiaire.

Selon un autre aspect, l'invention a pour objet l'utilisation telle que définie à la revendication 5.

La surface à traiter peut être en tout matériau et notamment en bois, en verre, en métal, par exemple en acier émaillé ou en acier inoxydable, en céramique, en polymère organique tel que par exemple les polyéthylènes, les polypropylènes, les polycarbonates, les polyamides, les polyesters tels que les polyéthylènes téréphtalates (PET), les polyuréthanes, les polymères fluorés tels que les PTFE, PVDF ou PFA, les polychlorures de vinyle (PVC) ; il peut aussi s'agir d'une surface peinte avec une peinture glycérophtalique, acrylique ou vinylique.

Dans un autre aspect préféré de la présente invention, la composition prête à l'emploi telle que décrite précédemment peut être utilisée pour désinfecter et détartrer le matériel mis en oeuvre à tous les stades de la chaîne de dialyse tels que notamment les circuits de traitement d'eau pour hémodialyse, et plus particulièrement la boucle de recirculation, les générateurs de dialyse, les dialyseurs, les hémofiltres, les hémodialyseurs ou les hémodiafiltres.

Dans un autre aspect préféré de la présente invention, la composition telle que définie précédemment est utilisée en hygiène hospitalière, notamment pour désinfecter le matériel médico-chirurgical réutilisable, tels que par exemple les endoscopes. La composition (C) telle que décrite précédemment est particulièrement appropriée à une telle utilisation.

De par sa stabilité et son innocuité, la composition telle que définie précédemment peut aussi être utilisée en milieu non hospitalier notamment, par le praticien en médecine libérale pour le nettoyage et la désinfection de son matériel de consultation réutilisable, comme par exemple le matériel de dentisterie ou les aiguilles d'acupuncture. Les compositions (A) ou (C) telles que décrites précédemment sont aussi particulièrement appropriées à une telle utilisation.

La composition B₁ telle que décrite précédemment est particulièrement appropriée à la désinfection des instruments médico-chirurgicaux tels que par exemple, les endoscopes lorsque celle-ci est réalisée en machines pour lavage-désinfection desdits instruments. Dans ce cas, la composition Bi est diluée entre le 1/3 et 1/10 et, plus particulièrement, au 1/5 lors de l'utilisation en machine.

Selon l'utilisation qui en est faite, la composition selon l'invention est mise en oeuvre de façon manuelle, semi-automatique ou automatique par exemple, par pulvérisation, par trempage, par badigeonnage, ou recirculation dans l'équipement dont on doit désinfecter les parois internes.

Dans un dernier aspect de la présente invention, la composition ou la solution diluée est absorbée sur un matériau poreux tel qu'une éponge, du papier, ou du tissu, pour former une formulation prête à l'emploi sur support solide telle que, par exemple, une lingette.

Les exemples suivants illustrent l'invention sans toutefois la limiter.

### A- PREPARATION DES COMPOSITIONS SELON L'INVENTION

Les compositions suivantes ont été préparées par addition de Génapol 2908D (II) et, soit de cocodiméthylamine (la) soit d'oxyde de tétradécyl diméthylamine (b) qui sont commercialisés sous la Marque Aromox, à une solution commerciale d'acide peracétique.

| | | | | | |
|---|---|---|---|---|---|
| **Compositions** | **APA % en poids** | **H**_{**2**}**O**_{**2**} **% en poids** | **AA % en poids** | **Composé de formule (I)% en poids** | **Composé de formule (II) % en poids** |
| 1 | 5,5 | 14,5 | 16,5 | (la) 0, 30 | 0,24 |
| 2 | 5,5 | 14,5 | 16,5 | (la) 0,20 | 0,24 |
| 3 | 5,5 | 14,5 | 16,5 | (la) 0,10 | 0,24 |
| 4 | 5,5 | 14,5 | 16,5 | (la) 0,05 | 0,24 |
| 5 | 5,5 | 14,5 | 16,5 | (la) 0,02 | 0,24 |
| 6 | 5,0 | 14,5 | 16,5 | (la) 0,30 | 0,24 |

A partir des compositions 1 à 6 selon l'invention, on a préparé, par dilution dans l'eau les compositions suivantes:

| Compositions | APA % en poids | (I) % en poids | pH |
|---|---|---|---|
| 1A invention | 0,050 | 0,0027 | 3,16 |
| 1B invention | 0,035 | 0,0019 | 3,29 |
| 1C invention | 0,020 | 0,0011 | 3,52 |
| 2A invention | 0,070 | 0,0025 | 2,99 |
| 2B invention | 0,055 | 0,0020 | 3,12 |
| 2C invention | 0,040 | 0,0014 | 3,26 |
| 3A invention | 0,080 | 0,0014 | 2,98 |
| 3B invention | 0,060 | 0,0011 | 3,11 |
| 3C invention | 0,040 | 0,0007 | 3,30 |
| 4A non revendiqué | 0,200 | 0,0017 | 2,59 |
| 4B non revendiqué | 0,170 | 0,0015 | 2,68 |
| 4C non revendiqué | 0,140 | 0,0012 | 2,77 |
| 4D non revendiqué | 0,100 | 0,0009 | 2,93 |
| 5A non revendiqué | 0,260 | 0,0012 | 2,45 |
| 5B non revendiqué | 0,240 | 0,0011 | 2,47 |
| 5C non revendiqué | 0,220 | 0,0010 | 2,50 |
| 5D non revendiqué | 0,200 | 0,0009 | 2,56 |
| 6A invention | 0,055 | 0,003 | 3,20 |

A l'exception de la solution 6A par laquelle le composé de formule (I) est le composé (Ib), toutes les autres solutions contiennent du composé (la).

### B - ANALYSE DES PROPRIETES FONGICIDES DES COMPOSITIONS SELON L'INVENTION

L'activité fongicide des compositions selon l'invention a été évaluée sur Candida parapsilosis selon la procédure AFNOR NFT 72 201 (temps de contact : 15 minutes; température 20°C). On obtient les abattements logarithmiques suivants :

| Compositions | Abattements log. |
|---|---|
| 1A | 6,9 |
| 1B | 6,7 |
| 2A | 6,2 |
| 2B | 6,2 |
| 2C | 5,4 |
| 3A | 6,3 |
| 4A | > 7 |
| 4B | > 7 |
| 4C | > 7 |
| 5A | > 7 |
| 5B | > 7 |
| 5D | > 7 |
| 6A | > 7 |

### C - ETUDE COMPARATIVE

On a évalué l'activité fongicide sur Candida parapsilosis selon la norme AFNOR NFT 72 201 (temps de contact : 15 minutes ; température 20°C) des solutions aqueuses suivantes :

| Solutions | APA % W/W | Acide acétique % W/W | Oxyde d'amine de formule (la) % W/W | pH |
|---|---|---|---|---|
| A | 0 | 2,5 | 0,003 | 2,62 |
| B | 0,29 | 0,9 | 0 | 2,25 |
| C | 0 | 2,5 | 0 | 2,62 |
| D | 0 | 0 | 0,008 | 6,67 |

On obtient les abatements logarithmiques suivants :

| Compositions | Abattements log. |
|---|---|
| A | 5,5 |
| B | 4,6 |
| C | 0 |
| D | 0 |

Les résultats montrent que :
- pour obtenir un abattement logarithmique de 4,6, il faut une concentration en APA, dans une solution sans oxyde d'amine, de 0,29 % (solution B) ;
- pour obtenir un abattement logarithmique de 5,5 , il faut en milieu acide, une concentration en oxyde d'amine, dans une solution sans acide peracétique, de 0,003 % (solution A) ;
- avec une solution contenant ensemble deux fois moins d'acide peracétique (0,14 %) et 2,5 fois moins d'oxyde d'amine (0,0012 %), on obtient un abattement logarithmique supérieur à 7 soit une activité fongicide environ 100 fois plus importante (solution 4C).

Les excellents résultats observés sont donc dus à la synergie induite par la présence de l'acide peracétique et d'un oxyde d'amine dans une même composition.

## Revendications

1. Composition (A) sous forme de solution aqueuse comprenant :
de 0,0005 % à 0,05 % en poids d'acide peracétique
de 0,01 % à 0,30 % en poids d'acide acétique,
de 0,3 % à 5 % en poids de peroxyde d'hydrogène,
de 0,0005 % à 0,01 % en poids d'au moins un composé de formule (I) et
de 0,0001 % à 0,005 % en poids d'au moins un composé de formule (II),
ou composition (B₁) sous forme de solution aqueuse comprenant :
de 0,25 % à 0,75 % en poids d'acide peracétique,
de 6 % à 10 % en poids de peroxyde d'hydrogène,
de 2 % à 10 % en poids d'acide acétique,
de 0,015 % à 0,75 % en poids de composé de formule (I) et
de 0,0001 % à 0,15 % en poids de composé de formule (II)
ou composition (C) sous forme d'une solution aqueuse comprenant :
de 0,05 % à 0,10 % en poids d'acide peracétique,
de 3 % à 4 % en poids de peroxyde d'hydrogène,
de 2 % à 3 % en poids d'acide acétique,
de 0,003 % à 0,006 % en poids de composé de formule (I),
de 0,001 % à 0,005 % en poids de composé de formule (II),
de 0,1 % à 0,8 % en poids de dihydrogénophosphate de sodium,
de 0,05 % à 0,1 % en poids de pyrophosphate de sodium et
de 0,0001 % à 0,05 % en poids d'un agent de coloration,
Compositions (A), (B₁) et (C) dans lesquelles le composé de formule (I) est représenté par la formule (I) :
(R₁)(R₂)(R₃)N→O (I)
dans laquelle, soit R₁ représente un radical, linéaire ou ramifié, cyclique ou acyclique, comportant de 1 à 40 atomes de carbone et éventuellement de 1 à 6 hétéroatomes choisis parmi l'oxygène, le soufre ou l'azote, et R₂ et R₃, identiques ou différents, représentent un radical alkyle, linéaire ou ramifié, comportant de 1 à 4 atomes de carbone ou un radical hydroxyalkyle, linéaire ou ramifié, comportant de 1 à 4 atomes de carbone ; soit R₁ et R₂ représentent ensemble avec l'atome d'azote auquel ils sont liés, un hétérocycle, saturé ou insaturé, substitué ou non substitué, comportant de 5 à 8 atomes de carbone et de 1 à 4 hétéroatomes choisis parmi l'oxygène, l'azote ou le soufre, et R₃ représente un radical alkyle, linéaire ou ramifié comportant de 1 à 4 atomes de carbone ou un radical hydroxyalkyle linéaire ou ramifié comportant de 1 à 4 atomes de carbone, dans lesquelles le composé de formule (II) est représentée par la formule (II) :
R₅-O-[CH(R₈)-CH(R₆)-O]ₙ-R₇ (II)
dans laquelle R₅ représente un radical aliphatique, linéaire ou ramifié, saturé ou insaturé, comportant de 5 à 31 atomes de carbone et de préférence de 10 à 16 atomes de carbone ; R₆ représente un atome d'hydrogène, un radical méthyle ou radical éthyle, R₈ représente un atome d'hydrogène, un radical méthyle ou radical éthyle, étant entendu qu'au moins un des deux radicaux R₆ ou R₈ représente un atome d'hydrogène, R₇ représente un atome d'hydrogène, ou un radical alkyle, linéaire ou ramifié comportant de 1 à 4 atomes de carbone, ou un radical benzyle, n représente un nombre compris entre 1 et 50 et, de préférence, n est inférieur à 20, étant entendu que dans les compositions (A), (B₁) et (C),
i) le rapport pondéral entre le composé de formule (I) et l'acide peracétique est inférieur ou égal à 0,2, et
ii) le rapport pondéral entre l'agent tensioactif non ionique de la formule (II) et l'acide péracétique est inférieur ou égal à 0,2,
et étant entendu que l'agent tensioactif non ionique présent dans la solution n'est ni un composé de formule (III)
(R₉)(R₁₀)CH-(O-CH₂-CH₂)ₙ-OH (III)
dans laquelle R₉ et R₁₀ représentent indépendamment l'un de l'autre un atome d'hydrogène ou un radical alkyle linéaire ou ramifié, le nombre total d'atomes de carbone dans R₉ plus R₁₀ étant compris entre 7 et 22, n est compris entre 1 et 15 et le rapport numérique entre le nombre total d'atomes de carbone dans R₉ plus R₁₀ et n est supérieur ou égal à 3/1, ni un nonyl phénol éthoxylate ayant un indice d'éthoxylation compris entre 4 et 8.

2. Composition telle que définie à la revendication 1, pour laquelle dans la formule (I), R₁ représente un radical aliphatique, linéaire ou ramifié, comportant de 8 à 18 atomes de carbone et R₂ et R₃ représentent chacun un radical méthyle et de préférence dans la formule (I), R₁ est choisi parmi les radicaux octyle, décyle, dodécyle, tétradécyle ou hexadécyle.

3. Composition telle que définie à l'une des revendications 1 ou 2 pour laquelle, dans la formule (II), R₇ représente un atome d'hydrogène et, soit le groupe [CH(R₈)-CH(R₆)-O]ₙ représente uniquement des motifs structuraux [CH₂-CH₂-O]ₙ, soit le groupe [CH(R₈)-CH(R₆)-O]ₙ, représente une chaîne constituée environ d'entre 5 et 8 motifs structuraux -(CH₂-CH₂₋O)- et environ d'entre 1 et 5 motifs structuraux -(CH₂CH(CH₃)-O), lesdits motifs structuraux étant distribués dans ladite chaîne de manière séquencée ou aléatoire.

4. Composition telle que définie à l'une quelconque des revendications 1 à 3 comprenant en outre l'un ou plusieurs des composés choisis parmi les agents de coloration, tels que les colorants comportant une fonction monoazo -N=N- ou les pigments de la famille des phtalocyanines, les agents stabilisants tels que les acides forts ou leurs sels alcalins, ou comportant en outre un agent inhibiteur de corrosion choisi parmi les sels de l'acide phosphorique et de préférence parmi le dihydrogénophosphate de sodium ou l'hydrogénophosphate de sodium.

5. Utilisation de la composition telle que définie à l'une des revendications 1 à 4 pour désinfecter les surfaces dures en hygiène hospitalière, notamment pour désinfecter le matériel médico-chirurgical.

6. Utilisation selon la revendication 5, en milieu non hospitalier, pour désinfecter le matériel de consultation du praticien de médecine libérale.

7. Utilisation d'une composition telle que définie à la revendication 1 pour désinfecter les équipements médicaux réutilisables et notamment les endoscopes.

8. Utilisation de la composition (B₁) telle que définie à la revendication 1, pour désinfecter, en machines pour lavage-désinfection des instruments médico-chirurgicaux, lesdits instruments tels que les endoscopes.

9. Utilisation selon l'une des revendications 5 ou 6, pour désinfecter et détartrer le matériel mis en oeuvre à tous les stades de la chaîne de dialyse tels que notamment les circuits de traitement d'eau, pour hémodialyse et plus particulièrement la boucle de recirculation, les générateurs de dialyse, les dialyseurs, les hémofiltres, les hémodialyseurs ou les hémodiafiltres.

## Patentansprüche

1. Zusammensetzung (A) in Form einer wässrigen Lösung, umfassend:
0,0005 Gew.-% bis 0,05 Gew.-% Peressigsäure
0,01 Gew.-% bis 0,30 Gew.-% Essigsäure
0,3 Gew.-% bis 5 Gew.-% Wasserstoffperoxid,
0,0005 Gew.-% bis 0,01 Gew.-% mindestens einer Verbindung der Formel (I) und
0,0001 Gew.-% bis 0,005 Gew.-% mindestens einer Verbindung der Formel (II),
oder Zusammensetzung (B₁) in Form einer wässrigen Lösung, umfassend:
0,25 Gew.-% bis 0,75 Gew.-% Peressigsäure
6 Gew.-% bis 10 Gew.-% Wasserstoffperoxid,
2 Gew.-% bis 10 Gew.-% Essigsäure
0,015% bis 0,75 Gew.-% der Verbindung der Formel (I) und
0,0001 Gew.-% bis 0,15 Gew.-% der Verbindung der Formel (II)
oder Zusammensetzung (C) in Form einer wässrigen Lösung, umfassend:
0,05 Gew.-% bis 0,10 Gew.-% Peressigsäure
3 Gew.-% bis 4 Gew.-% Wasserstoffperoxid,
2 Gew.-% bis 3 Gew.-% Essigsäure
0,003 Gew.-% bis 0,006 Gew.-% der Verbindung der Formel (I),
0,001 Gew.-% bis 0,005 Gew.-% der Verbindung der Formel (II),
0,1 Gew.-% bis 0,8 Gew.-% Natriumdihydrogenphosphat,
0,05 Gew.-% bis 0,1 Gew.-% Natriumpyrophosphat,
0,0001 Gew.-% bis 0,05 Gew.-% eines Färbemittels,
Zusammensetzungen (A), (B₁) und (C), wobei die Verbindung der Formel (I) für die Formel (I) steht:
(R₁) (R₂) (R₃)N→O (I)
in welcher R₁ entweder für einen linearen oder verzweigten zyklischen oder azyklischen Rest steht, der 1 bis 40 Kohlenstoffatome und eventuell 1 bis 6 Heteroatome umfasst, ausgewählt aus Sauerstoff, Schwefel oder Stickstoff, und R₂ und R₃, gleich oder verschieden, für einen linearen oder verzweigten Alkylrest stehen, der 1 bis 4 Kohlenstoffatome oder einen linearen oderverzweigten Hydroxyalkylrest umfasst, der 1 bis 4 Kohlenstoffatome umfasst; oder R₁ und R₂ zusammen mit dem Stickstoffatom, an das sie gebunden sind, für einen gesättigten oder ungesättigten, substituierten oder nicht substituierten Heterocyclus stehen, der 5 bis 8 Kohlenstoffatome und 1 bis 4 Heteroatome umfasst, ausgewählt aus Sauerstoff, Stickstoff oder Schwefel, und R₃ für einen linearen oder verzweigten Alkylrest steht, der 1 bis 4 Kohlenstoffatome oder einen linearen oder verzweigten Hydroxyalkylrest umfasst, der 1 bis 4 Kohlenstoffatome umfasst, in denen die Formel (II)
R₅-O-[CH(R₈)-CH(R₆)-O]ₙ-R₇ (II)
für die Verbindung der Formel (II) steht, in welcher R₅ für einen linearen oder verzweigten, gesättigten oder ungesättigten aliphatischen Rest steht, der 5 bis 31 Kohlenstoffatome und vorzugsweise 10 bis 16 Kohlenstoffatome enthält; R₆ für ein Wasserstoffatom, einen Methylrest oder einen Ethylrest steht, R₈ für ein Wasserstoffatom, einen Methylrest oder einen Ethylrest steht, wobei als vereinbart gilt, dass mindestens einer der zwei Reste R₆ oder R₈ für ein Wasserstoffatom steht, R₇ für ein Wasserstoffatom oder einen linearen oder verzweigten Alkylrest, der 1 bis 4 Kohlenstoffatome umfasst, oder für einen Benzylrest steht, n für eine Zahl zwischen 1 und 50 steht und n vorzugsweise nicht unter 20 liegt, wobei als vereinbart gilt, dass in den Zusammensetzungen (A), (B₁) und (C),
i) das Gewichtsverhältnis zwischen der Verbindung der Formel (I) und der Peressigsäure kleiner oder gleich 0,2 ist, und
ii) das Gewichtsverhältnis zwischen der nicht ionischen oberflächenaktiven Substanz der Formel (II) und der Peressigsäure kleiner oder gleich 0,2 ist,
und wobei als vereinbart gilt, dass die nicht ionische oberflächenaktive Substanz, die in der Lösung vorhanden ist, weder eine Verbindung der Formel (III)
(R₉) (R₁₀) CH-(O-CH₂-CH₂)ₙ-OH (III)
in welcher R₉ und R₁₀ unabhängig voneinander für ein Wasserstoffatom oder einen linearen oder verzweigten Alkylrest stehen, wobei die Gesamtzahl der Kohlenstoffatome in R₉ plus R₁₀ zwischen 7 und 22 liegt, n zwischen 1 und 15 und das Zahlenverhältnis zwischen der Gesamtzahl der Kohlenstoffatome in R₉ plus R₁₀ und n größer oder gleich 3/1 ist, noch ein Nonylphenol-ethoxylat mit einem Ethoxylierungsgrad zwischen 4 und 8 ist.

2. Zusammensetzung wie in Anspruch 1 definiert, für welche in Formel (I) R₁ für einen linearen oder verzweigten aliphatischen Rest steht, der 8 bis 18 Kohlenstoffatome umfasst, und R₂ und R₃ jeweils für einen Methylrest stehen und in der Formel (I) R₁ vorzugsweise ausgewählt wird aus einem Oktyl-, Decyl-, Dodecyl-, Tetradecyl- oder Hexadecylrest.

3. Zusammensetzung, wie in einem der Ansprüche 1 oder 2 definiert, für welche in der Formel (II) R₇ für ein Wasserstoffatom steht, und die Gruppe [CH(R₈)-CH(R₆)-O]ₙ entweder nur für die strukturellen Motive [CH₂-CH₂-O]ₙ steht, oder die Gruppe [CH(R₈)-CH(R₆)-O]ₙ für eine Kette steht, die ungefähr aus 5 bis 8 strukturellen Motiven -(CH₂-CH₂-O)- und ungefähr aus 1 bis 5 strukturellen Motiven -(CH₂₋CH(CH₃)-O) besteht, wobei die strukturellen Motive in der Kette aufeinander folgend oder zufällig verteilt sind.

4. Zusammensetzung wie in einem der Ansprüche 1 bis 3 definiert, die außerdem eine oder mehrere Verbindungen umfasst, ausgewählt aus den Färbemitteln, wie etwa den Farbstoffen, die eine Monoazo-Funktion -N=N- aufweisen, oder den Pigmenten der Familie der Phtalocyanine, den Stabilisierungsmitteln, wie etwa den starken Säuren oder deren Alkalisalze, oder die außerdem ein korrosionshemmendes Mittel umfasst, ausgewählt aus den Phosphorsäuresalzen und vorzugsweise aus Natriumdihydrogenphosphat oder Natriumhydrogenphosphat.

5. Verwendung der Zusammensetzung, wie in einem der Ansprüche 1 bis 4 definiert, um harte Oberflächen in der Krankenhaushygiene zu desinfizieren, besonders, um das medizinisch-chirurgische Material zu desinfizieren.

6. Verwendung nach Anspruch 5, außerhalb von Krankenhäusern, um die während der Sprechstunden verwendeten Geräte in freien Arztpraxen zu desinfizieren.

7. Verwendung einer ,Zusammensetzung, wie in Anspruch 1 definiert, um wieder verwendbare medizinische Ausrüstungsgegenstände und besonders Endoskope zu desinfizieren.

8. Verwendung der Zusammensetzung (B₁), wie in Anspruch 1 definiert, um medizinisch-chirurgische Instrumente in Wasch- und Desinfektionsmaschinen zu desinfizieren, wobei die Instrumente etwa Endoskope sind.

9. Verwendung nach einer der Ansprüche 5 oder 6, um das Material, das in allen Phasen des Dialyseprozesses eingesetzt wird, wie etwa besonders die Wasseraufbereitungskreisläufe, für die Hämodialyse und insbesondere den Umwälzkreislauf, die Dialysatwasseraufbereitungsgeräte, die Dialysatoren, die Hämofilter, die Hämodialysatoren oder die Hämodiafilter, zu desinfizieren und zu entkalken.

## Claims

1. Composition (A) in the form of aqueous solution, comprising:
from 0.0005% to 0.05% by weight of peracetic acid,
from 0.01% to 0.30% by weight of acetic acid,
from 0.3% to 5% by weight of hydrogen peroxide,
from 0.0005% to 0.01% by weight of at least one compound of formula (I) and
from 0.0001% to 0.005% by weight of at least one compound of formula (II),
or a composition (B₁) comprising
from 0.25% to 0.75% by weight of peracetic acid,
from 6% to 10% by weight of hydrogen peroxide,
from 2% to 10% by weight of acetic acid,
from 0.015% to 0.75% by weight of compound of formula (I) and
from 0.0001% to 0.15% by weight of a compound of formula (II)
or a composition (C) in the form of an aqueous solution comprising:
from 0.05% to 0.10% by weight of peracetic acid,
from 3% to 4% by weight of hydrogen peroxide,
from 2% to 3% by weight of acetic acid,
from 0.003% to 0.006% by weight of a compound of formula (I),
from 0.001% to 0.005% by weight of a compound of formula (II),
from 0.1% to 0.8% of sodium dihydrogen phosphate,
from 0.05% to 0.1% by weight of sodium pyrophosphate and
from 0.0001% to 0.05% by weight of a colouring agent,
Compositions (A), (B) and (C) in which the compound of formula (I) is represented by formula (I) :
(R₁) (R₂) (R₃)N → O (I)
in which, either R₁ represents a linear or branched, cyclic or acyclic radical, containing from 1 to 40 carbon atoms and optionally from 1 to 6 heteroatoms chosen from oxygen, sulphur or nitrogen, and R₂ and R₃, which are identical or different, represent a linear or branched alkyl radical containing from 1 to 4 carbon atoms or a linear or branched hydroxyalkyl radical containing from 1 to 4 carbon atoms; or R₁ and R₂ represent, together with the nitrogen atom to which they are attached, a saturated or unsaturated, substituted or unsubstituted heterocycle containing from 5 to 8 carbon atoms and from 1 to 4 heteroatoms chosen from oxygen, nitrogen or sulphur, and R₃ represents a linear or branched alkyl radical containing from 1 to 4 carbon atoms or a linear or branched hydroxyalkyl radical containing from 1 to 4 carbon atoms, in which the compound of formula (II) is represented by the formula (II):
R₅-O-[CH(R₈)-CH(R₆)-O]ₙ-R₇ (II)
in which R₅ represents a linear or branched, saturated or unsaturated aliphatic radical containing from 5 to 31 carbon atoms and preferably from 10 to 16 carbon atoms; R₆ represents a hydrogen atom, a methyl radical or an ethyl radical, R₈ represents a hydrogen atom, a methyl radical or an ethyl radical, it being understood that at least one of the two radicals R₆ or R₈ represents a hydrogen atom, R₇ represents a hydrogen atom, or a linear or branched alkyl radical containing from 1 to 4 carbon atoms, or a benzyl radical, n represents a number between 1 and 50 and, preferably, n is less than 20, it being understood that in the compositions (A), (B), and (C),
i) the weight ratio between the compound of formula (I) and the peracetic acid is less than or equal to 0.2, and
ii) the weight ratio between the nonionic surfactant of the formula (II) and the peracetic acid is less than or equal to 0.2,
and it being understood that the nonionic surfactant present in the solution is neither a compound of formula (III)
(R₉)(R₁₀)CH-(O-CH₂-CH₂)ₙ-OH (III)
in which R₉ and Rio represent, independently of each other, a hydrogen atom or a linear or branched alkyl radical, the total number of carbon atoms in R₉ plus R₁₀ being between 7 and 22, n is between 1 and 15 and the numerical ratio between the total number of carbon atoms in R₉ plus R₁₀ and n is greater than or equal to 3/1, nor a nonylphenol ethoxylate having an ethoxylation value of between 4 and 8.

2. Composition as defined in Claim 1, for which, in formula (I), R₁ represents a linear or branched aliphatic radical containing from 8 to 18 carbon atoms and R₂ and R₃ each represent a methyl radical and preferably, in formula (I), R₁ is chosen from the octyl, decyl, dodecyl, tetradecyl or hexadecyl radicals.

3. Composition as defined in either of Claims 1 and 2, for which, in formula (II), R₇ represents a hydrogen atom and, either the group [CH(R₈)-CH(R₆)-O]ₙ represents only structural units [CH₂₋CH₂-O]ₙ, or the group [CH(R₈)-CH(R₆)-O]ₙ represents a chain consisting of approximately between 5 and 8 structural units -(CH₂-CH₂-O)- and approximately between 1 and 5 structural units -(CH₂-CH(CH₃)-O), the said structural units being distributed in the said chain sequentially or randomly.

4. Composition as defined in any one of Claims 1 to 3, comprising, in addition, one or more of the compounds chosen from colouring agents such as colorants containing a monoazo functional group -N=N- or pigments of the phthalocyanine family, stabilizing agents such as strong acids or their alkaline salts, or containing in addition a corrosion inhibitor chosen from phosphoric acid salts and preferably from sodium dihydrogen phosphate or sodium hydrogen phosphate.

5. Use of the composition as defined in one of Claims 1 to 4, for disinfecting hard surfaces in hospital hygiene, in particular for disinfecting medical and surgical equipment.

6. Use according to Claim 5, in a non-hospital environment, for disinfecting the equipment used for consultation by practitioners of liberal medicine.

7. Use of a composition as defined in Claim 1, for disinfecting reusable medical equipment and in particular endoscopes.

8. Use of composition (B₁) as defined in Claim 1, for disinfecting, in machines for washing-disinfecting medical and surgical instruments, the said instruments such as endoscopes.

9. Use according to one of Claims 5 or 6, for disinfecting and descaling the equipment used at all stages of the dialysis chain, such as in particular the water treatment circuits, for haemodialysis and more particularly the recirculation loop, the dialysis generators, the dialysers, the haemofilters, the haemodialysers or the haemodiafilters.
